# EUROPÄISCHE PATENTSCHRIFT

(11) **EP 2 276 831 B1**
(45) Veröffentlichungstag und Bekanntmachung des Hinweises auf die Patenterteilung: **05.09.2012**
(21) Anmeldenummer: 09737819.4
(22) Anmeldetag: 16.04.2009
(51) Int. Cl.: C12N 1/20

(54) **ZUSAMMENSETZUNG ZUR KULTIVIERUNG VON ANSPRUCHSVOLLEN BAKTERIEN**
COMPOSITION FOR CULTIVATING SOPHISTICATED BACTERIA
COMPOSITION POUR LA CULTURE DE BACTÉRIES EXIGEANTES

(30) Priorität: 29.04.2008 DE 102008022333
(43) Veröffentlichungstag der Anmeldung: 26.01.2011
(73) Patentinhaber: Eberhard-Karls-Universität Tübingen Universitätsklinikum, 72076 Tübingen (DE)
(72) Erfinder: KEMPF, Volkhard, 63303 Dreieich-Buchschlag (DE)
(74) Vertreter: Witte, Weller & Partner
(86) Internationale Anmeldenummer: PCT/EP2009/002790
(87) Internationale Veröffentlichungsnummer: WO 2009/132775

(56) Entgegenhaltungen:
- SCHWARTZMAN W A ET AL: "Development and evaluation of a blood-free medium for determining growth curves and optimizing growth of Rochalimaea henselae." JOURNAL OF CLINICAL MICROBIOLOGY JUL 1993, Bd. 31, Nr. 7, Juli 1993 (1993-07), Seiten 1882-1885, XP002536815 ISSN: 0095-1137
- MAGGI RICARDO G ET AL: "Novel chemically modified liquid medium that will support the growth of seven bartonella species." JOURNAL OF CLINICAL MICROBIOLOGY JUN 2005, Bd. 43, Nr. 6, Juni 2005 (2005-06), Seiten 2651-2655, XP002536816 ISSN: 0095-1137
- JONES ET AL: "Use of an insect cell culture growth medium to isolate bacteria from horses with effusive, fibrinous pericarditis: A preliminary study" VETERINARY MICROBIOLOGY, ELSEVIER BV, NL, Bd. 121, Nr. 1-2, 28. Februar 2007 (2007-02-28), Seiten 177-181, XP005914469 ISSN: 0378-1135
- RIESS TANJA ET AL: "Analysis of a novel insect cell culture medium-based growth medium for Bartonella species." APPLIED AND ENVIRONMENTAL MICROBIOLOGY AUG 2008, Bd. 74, Nr. 16, August 2008 (2008-08), Seiten 5224-5227, XP002536817 ISSN: 1098-5336

## Beschreibung

Die vorliegende Erfindung betrifft eine Zusammensetzung zur Kultivierung von anspruchsvollen Bakterien, vorzugsweise der Gattung *Bartonella,* sowie ein Verfahren zur Kultivierung dieser Bakterien.

Die Kultivierung anspruchsvoller Bakterien stellt ein bekanntes Problem der Mikrobiologie und der medizinischen Diagnostik dar. Ein derartiges anspruchsvolles Bakterium gehört zu der Gattung *Bartonella.* Bartonellen sind wichtige Pathogene der Human- und Veterinärmedizin. Gegenwärtig werden *Bartonella henselae* und *Bartonella quintana* für die relevantesten humanpathogenen *Bartonella-Species* gehalten, die Krankheiten wie die Katzenkratzkrankheit, bazilläre Angiomathose, Schützengrabenfieber und viele weitere verursachen, vgl. Dehio, C. (2005), Bartonella-host-cell interactions and vascular tumour formation. Nat. Rev. Microbiol. 3:621-631. Vor kurzem wurden neue Pathogene mit unklarer Epidemiologie beschrieben, z.B. *Bartonella rochalimeae;* vgl. Eremeeva, M.E. et al. (2007), Bacteremia, fever and spenomegaly caused by a newly recognized Bartonella species. N. Engl. J. Med. 256:2381-2387. *Bartonella* spp., z.B. *Bartonella henselae, Bartonella vinsonii* subsp. *berkhoffii, Bartonella schoenbuchiensis* und viele andere finden sich in einem breiten Spektrum von Säugetieren, einschließlich Katzen, Hunden, Wiederkäuern und Nagetieren, die entweder unter diesen Infektionen leiden oder als asymptomatische Reservoirwirte für Zoonosen dienen.

Es ist bekannt, dass sich Bakterien der Gattung *Bartonella* nur sehr schlecht kultivieren lassen. Die Diagnostik von Bartonella-Infektionen erfolgt deshalb üblicherweise unter Verwendung von serologischen Verfahren oder durch molekulare Methoden, z.B. über die Amplifikation der 16S-rDNA; vgl. Centers of Disease Control and Prevention (CDC) (1999), Serodiagnosis of Emerging Infectious Diseases: *Bartonella* and *Ehrlichia* Infections (course manual), und Dauga, C. et al. (1996), Identification of Bartonella henselae and B. quintana 16s rDNA sequences by branch-, genus- and species-specific amplification. J. Med. Microbiol. 45:192-199.

*Bartonella* spp. werden üblicherweise kultiviert, indem hochsupplementierter Blutagar, z.B. Schokoladenagar, Schafsblutagar oder Kochblutagar, verwendet wird. Die Verwendung dieser Agars hat den Nachteil, dass die Inkubationszeiten sehr lang sind, nämlich bis zu 45 Tagen, so dass es zu Kontaminationsproblemen kommt; vgl. Maurin, M. (1994), Isolation and characterization by immunofluorescence, sodium dodecyl sulfate-polyacrylamide gel electrophoresis, western blot, restriction fragment length polymorphism-PCR, 16S rRNA gene sequencing, and pulsed-field gel electrophoresis of Rochalimaea quintana from a patient with bacillary angiomatosis. J. Clin. Microbiol. 32:1166-1171. Bis heute limitiert das langsame Wachstum auf festen Agar-basierenden Medien Experimente zur molekularen Analyse der Pathogenität von *Bartonella henselae.*

In der WO 03/012058 wird ein Medium zur Kultivierung von anspruchsvollen Mikroorganismen, wie *Bartonella* spp., beschrieben. Dieses Medium hat jedoch ebenfalls den Nachteil, dass die Mikroorganismen nur ein sehr langsames Wachstum zeigen. Hinzu kommt, dass das Medium aus 20 Einzelsubstanzen besteht, was die Herstellung sehr kompliziert und störanfällig macht. Das Medium erfordert den Zusatz von Schafsblut, was die Suspension trüb macht und deshalb keine Ermittlung der optischen Dichten erlaubt. Der Einsatz dieses Mediums im Rahmen einer automatisierten Messung des Wachstums der anspruchsvollen Bakterien ist deshalb kaum möglich, da diese Messung i.d.R. auf der Erfassung der optischen Dichten der Kulturen basiert. Eine entsprechende Limitierung besteht deshalb bei Untersuchungen zur Empfindlichkeit der anspruchsvollen Bakterien gegenüber Antibiotika, da auch dort meist optische Messungen vorgenommen werden.

Schwartzman et al. (1993) beschreiben die Kultivierung von *Bartonella henselae* in einem mit Agar versetzten Kultivierungsmedium, das als "Brucella Broth" bezeichnet wird und dem Fetales Kälberserum (FCS) hinzugesetzt wurde.

Maggi et al. (2005) beschreiben die Kultivierung von Bakterien der Gattung *Bartonella* in einem als BAPGM bezeichneten Medium, das auf dem Insektenzellmedium DS2 basiert.

Jones et al. (2007) beschreiben die Kultivierung verschiedener Bakterien auf Agarplatten, die 90 % des Insektenzellmediums DS2 und 10 % Schafsblut enthalten.

Vor diesem Hintergrund ist es eine Aufgabe der vorliegenden Erfindung, eine neue Zusammensetzung zur Kultivierung von Bakterien der Gattung *Bartonella* bereitzustellen, mit dem die Nachteile aus dem Stand der Technik vermieden werden. Insbesondere soll ein Flüssigkulturmedium bereitgestellt werden, das ein rasches Wachstum der anspruchsvollen Bakterien ermöglicht und dabei kostengünstig herstellbar ist.

Diese Aufgabe wird durch die Bereitstellung einer Zusammensetzung gelöst, die modifiziertes Schneider's Drosophila Medium (Schneider's Drosophila Medium revised) und Fetales Kälberserum (FCS) aufweist.

Die Erfinder haben überraschenderweise herausgefunden, dass sich die anspruchsvollen Bakterien besonders rasch und gut in dem Medium kultivieren lassen, das bislang zur Kultivierung von Insektenzellen verwendet wurde, dem FCS zugesetzt wird.

Diese Erkenntnis war überraschend. So wird im Stand der Technik zwar beschrieben, dass *Bartonella henselae* schneller wächst, wenn es mit Wirtszellen coinkubiert wird; vgl. Kempf, V.A. et al. (2000), Interaction of Bartonella henselae with endothelial cells results in rapid bacterial rRNA synthesis and replication. Cell. Microbiol. 2:431-441; La Scola, B. und D. Raoult (1999), Culture of Bartonella quintana and Bartonella henselae from human samples: a 5-year experience (1993 to 1998). J. Clin. Microbiol. 37:1899-1905; und Weiss, E. et al. (1978), Vole agent identified as a strain of the trench rickettsia, Rochalimaea Quintana. Infect. Immun. 19:1013-1020. Die Autoren haben jedoch nicht erkannt, dass die anspruchsvollen Bakterien auch in Abwesenheit von Wirtszellen in Insektenzellkulturmedium unter Zusatz von FCS wachsen.

Erfindungsgemäß wird unter einer "Zusammensetzung zur Kultivierung" ein Kultur- bzw. Kultivierungsmedium für biologisches Material verstanden. Das Kultivierungsmedium kann dazu verwendet werden, um anspruchsvolle Bakterien vital zu halten, wachsen zu lassen und/oder zu isolieren. Bei dem Kultivierungsmedium kann es sich um ein flüssiges, halbflüssiges oder festes Kultivierungsmedium handeln, wobei ein flüssiges Medium bevorzugt ist.

"Anspruchsvolle Bakterien" sind solche Bakterien, die persistent gegenüber einer Kultivierung in konventionellen Medien sind, wie beispielsweise LB-Medium, Dulbecco's modified Eagle's medium, F12-medium und dergleichen. Sie werden deshalb bislang in hochsupplementierten Blutagars, wie z.B. Schokoladenagar, Schafsblutagar oder Kochblutagar, kultiviert. Auch in diesen Medium zeigen die anspruchsvollen Bakterien nur ein sehr langsames Wachstum. Anspruchsvolle Bakterien erfordern stringentere Bedingungen im Hinblick auf die chemische Zusammensetzung der Kultivierungsmedien als nicht-anspruchsvolle bzw. anspruchslose Bakterien. Beispielsweise erfordern anspruchsvolle Bakterien, dass bestimmte organische Moleküle in dem Medium bereitgestellt werden, da diese eine defekte oder unzureichende enzymatische Maschinerie für die Synthese von Nährstoffen aufweisen, wie beispielsweise Vitaminen, Aminosäuren, Lipiden, Cofaktoren, wie NAD und NADH, Nukleosiden oder Fängermoleküle für freie Radikale etc. Alternativ oder zusätzlich kann das anspruchsvolle Bakterium erfordern, dass bestimmte organische Moleküle in dem Medium bereitgestellt werden, oder aufgrund einer Intoleranz des Bakteriums für bestimmte Komponenten, z.B. Zucker, in dem Medium fehlen.

Das anspruchsvolle Bakterium kann ein aufgrund von Stress nach der Induktion in dem Wirt defektes oder geschädigtes Bakterium sein, z.B. als Ergebnis eines antibakteriellen Mechanismus durch den Wirt, Antibiotika oder Surfactantproduktion durch die Lunge; diese Bakterien werden auch als immunologisch geschädigte Bakterien bezeichnet. Beispielsweise kann das Bakterium eine Schädigung der Zellwand oder der Zellwandsynthese aufweisen. Zusätzlich können Transportmechanismen oder zelluläre metabolische Wege als Ergebnis einer Schädigung oder Stress für das Bakterium gestört sein. Alternativ kann es sich bei dem anspruchsvollen Bakterium um ein lebensfähiges, jedoch nach den im Stand der Technik bekannten Methoden nicht kultivierbares Bakterium handeln, das eine metabolisch inaktive Form eines kultivierbaren Bakteriums darstellt. Defekte Bakterien, die Stress ausgesetzt sind oder metabolisch inaktive Bakterien, sind resistenter oder widerstandfähiger gegenüber Standardkultivierungs- und Isolierungstechniken. Alternativ kann es sich bei anspruchsvollen Bakterien um solche handeln, die sich an die Umgebung des Wirtes angepasst haben und keine Kultivierungsbedingungen mehr tolerieren, die wesentlich von den Bedingungen innerhalb des Wirtes abweichen.

Beispiele für anspruchsvolle Bakterien sind solche der Gattung *Bartonella, Bordetella* spp., *Borrelia* spp., *Anaplasma* spp. und *Mycobacterium* spp.

"Insektenzellkulturmedium" ist ein solches Kultivierungsmedium, das im Stand der Technik zur Kultivierung von Insektenzellen vorgesehen ist. Beispiele für solche Medien sind Schneider's Medium, Schneider's modifiziertes Serum ("Schneider's Drosophila revised"), Grace's Medium, IPL-41-Medium, TMN-FH-Medium und TC-100-Medium.

"Fetales Kälberserum" (FCS; engl. "fetal calf serum"), das auch als fetales Rinderserum (FBS; engl. "fetal bovine serum") oder Neugeborenen Kälberserum (NCS; engl. "newborn calf serum") bezeichnet wird, ist eine flüssige Zusammensetzung, die aus dem Blut von Kuhfeten gewonnen wird. Es enthält eine Vielzahl von Proteinen, von denen heute noch nicht alle bekannt sind. Hauptbestandteil ist "Rinderserumalbumin" oder "BSA" (engl. für "bovine serum albmin"), ein globuläres Protein, das aus 584 Aminosäuren besteht und eine relativen Molekülmasse von ca. 64.500 aufweist. Weitere Proteine stellen z.B. Wachstumsfaktoren dar. Es bildet die Hauptkomponente von Fetalem Kälberserum (FCS).

Die der Erfindung zugrunde liegende Aufgabe wird durch die Bereitstellung einer Zusammensetzung, die modifiziertes Schneider's Drosophila Medium und FCS aufweist, vollkommen gelöst. Das von den Erfindern verwendete Medium ist einfach herzustellen und erlaubt ein relativ schnelles planktonisches Wachstum der anspruchsvollen Bakterien.

Das erfindungsgemäß verwendete Medium stellt deshalb auch eine Voraussetzung zur Entwicklung von raschen diagnostischen Verfahren zum Nachweis von pathogenen anspruchsvollen Bakterien dar. Auch die molekulare Analyse der Pathogenität anspruchsvoller Bakterien wird nun verbessert möglich. Ein weiterer Vorteil der erfindungsgemäß verwendeten Zusammensetzung besteht darin, dass diese kein Hämin enthält, da bekannt ist, dass bestimmte anspruchsvolle Bakterien, wie solche der Gattung *Bartonella,* nur bestimmte Häminkonzentrationen tolerieren; vgl. Schwartzman, W.A. et al. (1993), Development and evaluation of a blood-free medium for determining growth curves and optimizing growth of Rochalinaea henselae. J. Clin. Microbiol. 31:1882-1885.

Bei den anspruchsvollen Bakterien handelt es um solche der Gattung *Bartonella.*

Diese Maßnahme hat den Vorteil, dass eine solche Zusammensetzung bereitgestellt wird, mit einer besonders anspruchsvollen Bakterienspezies, die in eine Vielzahl von Human- und Veterinärerkrankungen involviert ist.

Bei dem Insektenzellkulturmedium handelt es sich um modifiziertes Schneider's Drosophila-Medium.

Diese Maßnahme hat den Vorteil, dass ein solches Insektenzellkulturmedium Verwendung findet, das nach Erkenntnissen der Erfinder besonders geeignet ist. Die Zusammensetzung von modifiziertem Schneider's Drosophila-Medium ist beispielsweise offenbart in Schneider, I. und A. Blumenthal (1978), Drosophila cell and tissue culture. In: Biology and genetics of Drosophila, Vol. 2A, M. Ashburner und T.R.F. Wright Hsg., Academic Press, New York, Seiten 266-315, insbesondere Seite 308; und in Schneider, I. (1972), Cell lines derived from late embryonic stages of Drosophila melanogaster. J. Embryol. Exp. Morphol. 27: 353-365; und in der Produktinformation S 0146 "Schneider's insect medium" SIGMA-Aldrich, St. Louis, Missouri, USA; sowie der Produktinformation "Schneider Drosophila, revised", SERVA Electrophoresis GmbH, Heidelberg, Deutschland; Kat.-Nr. 47521.

Offenbart wird eine Zusammensetzung, die folgende Bestandteile aufweist:

| Anorganische Salze, nämlich | | |
|---|---|---|
| | CaCl₂ (wasserfrei) [in mg/l H₂O]: | ca. 50 bis ca. 2000, |
| | KCl [in mg/l H₂O]: | ca. 200 bis ca. 3000, |
| | KH₂PO₄ [in mg/l H₂O]: | ca. 30 bis ca. 1500, |
| | MgSO₄·7H₂O [in mg/l H₂O]: | ca. 1000 bis ca. 6000, |
| | NaCl [in mg/l H₂O]: | ca. 300 bis ca. 4000, |
| | NaHCO₃ [in mg/l H₂O]: | ca. 25 bis ca. 1300, |
| | Na₂HPO₄ [in mg/l H₂O]: | ca. 60 bis ca. 2500, |
| | | |

| Aminosäuren, nämlich | | |
|---|---|---|
| | β-Alanin [in mg/l H₂O] | ca. 30 bis ca. 1800, |
| | L-Arginin [in mg/l H₂O]: | ca. 50 bis ca. 2000, |
| | L-Asparaginsäure [in mg/l H₂O]: | ca. 25 bis ca. 1300, |
| | L-Cystin [in mg/l H₂O]: | ca. 5 bis ca. 200, |
| | L-Cystin·2HCl [in mg/l H₂O]: | ca. 2 bis ca. 150, |
| | L-Glutamin [in mg/l H₂O]: | ca. 250 bis ca. 3500, |
| | L-Glutaminsäure [in mg/l H₂O]: | ca. 75 bis ca. 2600, |
| | Glycin [in mg/l H₂O]: | ca. 20 bis ca. 1000, |
| | L-Histidin [in mg/l H₂O]: | ca. 25 bis ca. 1300, |
| | L-Isoleucin (in mg/l H₂O]: | ca. 10 bis ca. 400, |
| | L-Leucin [in mg/l H₂O]: | ca. 10 bis ca. 400, |
| | L-Lysin·HCl [in mg/l H₂O]: | ca. 200 bis ca. 3000, |
| | L-Methionin [in mg/l H₂O]: | ca. 10 bis ca. 400, |
| | L-Prolin [in mg/l H₂O]: | ca. 250 bis ca. 3500, |
| | L-Serin [in mg/l H₂O]: | ca. 20 bis ca. 1000, |
| | L-Threonin [in mg/l H₂O]: | ca. 25 bis ca. 1300, |
| | L-Tryptophan [in mg/l H₂O]: | ca. 10 bis ca. 400, |
| | L-Tyrosin [in mg/l H₂O]: | ca. 30 bis ca. 1800, |
| | L-Valin [in mg/l H₂O]: | ca. 20 bis ca. 1000, |
| | | |

| andere Bestandteile, nämlich | | |
|---|---|---|
| | Apfelsäure [in mg/l H₂O]: | ca. 5 bis ca. 200, |
| | Bernsteinsäure [in mg/l H₂O]: | ca. 50 bis ca. 2000, |
| | Fumarinsäure [in mg/l H₂O]: | ca. 5 bis ca. 200, |
| | Glucose [in mg/l H₂O]: | ca. 300 bis ca. 4000, |
| | Hefe-Extrakt [in mg/l H₂O]: | ca. 300 bis ca. 4000, |
| | α-Ketoglutarsäure [in mg/l H₂O]: | ca. 25 bis ca. 1300, |
| | α-Trehalose [in mg/l H₂O]: | ca. 300 bis ca. 4000, und |
| Fetales Kälberserum (FCS) [in Vol.-%] | | ca. 1 bis ca. 25. |

Diese Maßnahme hat den Vorteil, dass die wichtigsten Parameter der erfindungsgemäßen Zusammensetzung bereitgestellt werden und dem Fachmann eine einfache Herstellung ermöglichen.

Nach einer bevorzugten Ausgestaltung weist die erfindungsgemäß verwendete Zusammensetzung folgende Bestandteile auf:

| Anorganische Salze, nämlich | | |
|---|---|---|
| | CaCl₂ (wasserfrei) [in mg/l H₂O]: | 600, |
| | KCl [in mg/l H₂O]: | 1600, |
| | KH₂PO₄ [in mg/l H₂O]: | 450, |
| | MgSO₄·7H₂O [in mg/l H₂O]: | 3700, |
| | NaCl [in mg/l H₂O]: | 2100, |
| | NaHCO₃ [in mg/l H₂O]: | 400, |
| | Na₂HPO₄ [in mg/l H₂O]: | 700, |
| | | |

| Aminosäuren, nämlich | | |
|---|---|---|
| | β-Alanin [in mg/l H₂O]: | 500, |
| | L-Arginin [in mg/l H₂O]: | 600, |
| | L-Asparaginsäure [in mg/l H₂O]: | 400, |
| | L-Cystein [in mg/l H₂O]: | 60, |
| | L-Cystein·2HCl [in mg/l H₂O]: | 21,7, |
| | L-Glutamin [in mg/l H₂O]: | 1800, |
| | L-Glutaminsäure [in mg/l H₂O]: | 800, |
| | Glycin [in mg/l H₂O]: | 250, |
| | L-Histidin [in mg/l H₂O]: | 400, |
| | L-Isoleucin [in mg/l H₂O]: | 150, |
| | L-Leucin [in mg/l H₂O]: | 150, |
| | L-Lysin·HCl [in mg/l H₂O]: | 1650, |
| | L-Methionin [in mg/l H₂O]: | 150, |
| | L-Prolin [in mg/l H₂O]: | 1750, |
| | L-Serin [in mg/l H₂O]: | 250, |
| | L-Threonin [in mg/l H₂O]: | 350, |
| | L-Tryptophan [in mg/l H₂O]: | 100, |
| | L-Tyrosin [in mg/l H₂O]: | 500, |
| | L-Valin [in mg/l H₂O]: | 300, |
| | | |

| andere Bestandteile, nämlich | | |
|---|---|---|
| | Apfelsäure [in mg/l H₂O]: | 60, |
| | Bernsteinsäure [in mg/l H₂O]: | 600, |
| | Fumarinsäure [in mg/l H₂O]: | 60, |
| | Glucose [in mg/l H₂O]: | 2000, |
| | Hefe-Extrakt [in mg/l H₂O]: | 2000, |
| | α-Ketoglutarsäure [in mg/l H₂O]: | 350, |
| | α-Trehalose [in mg/l H₂O]: | 2000, und |
| | | |
| Fetales Kälberserum (FCS) [in Vol.-%] | | 10. |

Diese Maßnahme hat den Vorteil, dass die genauen Konzentrationen der erfindungsgemäß verwendeten Zusammensetzung bereitgestellt werden, die nach Erkenntnissen der Erfinder besonders gute Ergebnisse liefern.

Weiter ist es bevorzugt, wenn die Zusammensetzung zusätzlich 0,5 bis 50% (w/v), weiter bevorzugt 5% (w/v) Saccharose aufweist.

Wie die Erfinder feststellen konnten, wachsen anspruchsvolle Bakterien der Gattung *Bartonella* in der erfindungsgemäß verwendeten Zusammensetzung noch besser, wenn dieser Saccharose in den angegebenen Konzentrationsbereichen zugesetzt wird. Überraschenderweise führt beispielsweise der Zusatz anderer Kohlenhydrate, wie Mannose oder Galactose, sogar zu einer Verschlechterung des Wachstums der Bakterien.

Weiter ist es bevorzugt, wenn die erfindungsgemäß verwendete Zusammensetzung einen pH-Wert aufweist, der bei 5,5 bis 7,5, weiter bevorzugt bei 6,5 liegt.

Diese Maßnahme hat den Vorteil, dass bei dem angegebenen pH-Wert eine besonders gute Löslichkeit der Bestandteile gegeben ist und beispielsweise Ausfällungen weitgehend vermieden werden. Dies trifft insbesondere bei einem pH-Wert von 6,5 zu.

Weiter ist es bevorzugt, wenn die erfindungsgemäß verwendete Zusammensetzung sterilisiert ist.

Diese Maßnahme hat den Vorteil, dass Kontaminationen bei der Verwendung der Zusammensetzung, beispielsweise durch nicht-anspruchsvolle Bakterien, vermieden werden.

Ein weiterer Gegenstand der vorliegenden Erfindung betrifft ein Verfahren zur Kultivierung von Bakterien der Gattung *Bartonella,* das folgende Schritte aufweist, (1) Bereitstellung einer Zusammensetzung, (2) Inokulieren der Zusammensetzung mit Bakterien der Gattung *Bartonella,* vorzugsweise mit 1 x 10⁵ bis 1 x 10⁶ Bakterienzellen, (3) Inkubation der Zusammensetzung mit den Bakterien unter Kultivierungsbedingungen, vorzugsweise für 1 bis 20 Tage und vorzugsweise bei 37°C und vorzugsweise in einer Atmosphäre mit 5 Vol.-% CO₂, wobei als Zusammensetzung die Zusammensetzung der vorstehend beschriebenen erfindungsgemäßen Verwendung verwendet wird.

Die Erfinder stellen hiermit ein besonders geeignetes Verfahren zur Kultivierung von anspruchsvollen Bakterien bereit, mit dem in kürzester Zeit eine Bakterienkultur gewonnen werden kann.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen näher erläutert, aus denen sich weitere Eigenschaften und Vorteile ergeben. Die Ausführungsbeispiele schränken die Reichweite der Erfindung nicht ein. Dabei wird Bezug genommen auf die beigefügten Figuren, auf denen Folgendes zu sehen ist:

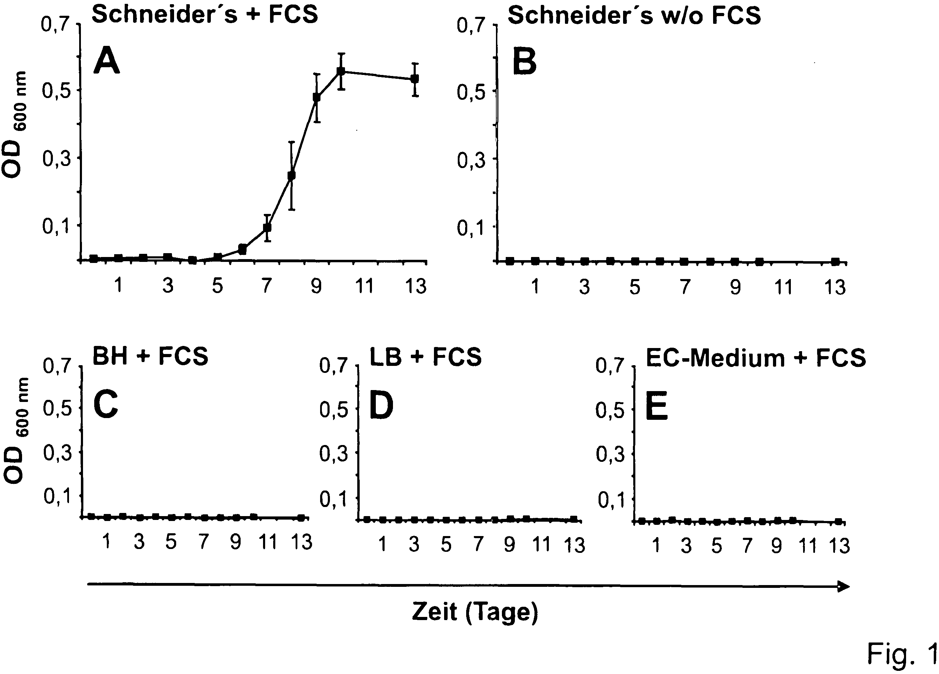

- Fig. 1:: Wachstumskurven von *Bartonella henselae* in verschiedenen Flüssigkeitswachstumsmedien. Verschiedene Wachstumsmedien wurden mit *Bartonella henselae* (1 x 10⁵/ml) inokuliert und das Bakterienwachstum wurde bestimmt, indem in 24-stündigen Intervallen die optische Dichte (OD_{600 nm}) gemessen wurde. (A) Schneider's Medium mit 10 Vol.-% FCS, (B) reines Schneider's Medium, (C) BH-Medium mit 10 Vol.-% FCS, (D) LB-Medium mit 10 Vol.-% FCS, (E) Endothelzellen-Wachstumsmedium (EC) mit 10 Vol.-% FCS. Festzustellen ist, dass das Wachstum von *Bartonella henselae* nur in Schneider's Medium beobachtet wird, das mit 10 Vol.-% FCS versetzt war.

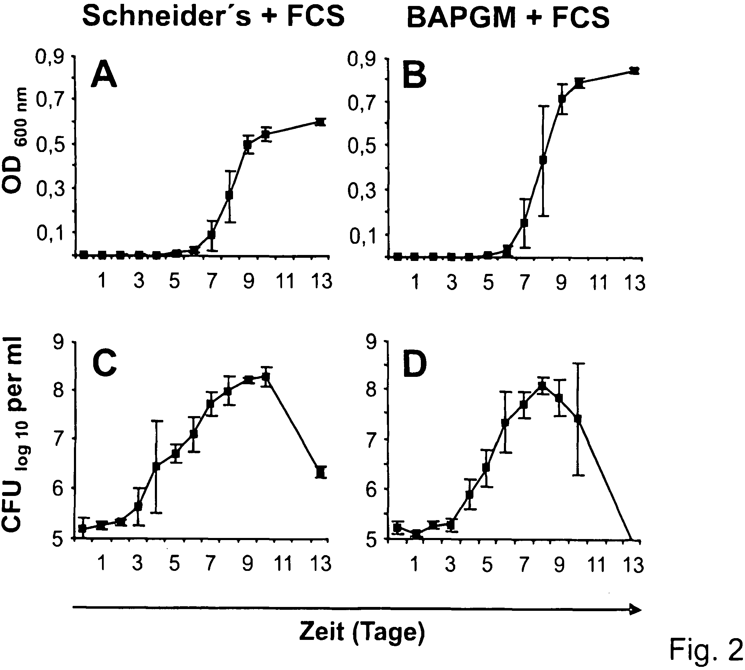

- Fig. 2:: Wachstumskurven von *Bartonella henselae* in Schneider's Medium und BAPGM. Sowohl Schneider's Medium als auch BAPGM wurden mit 10% FCS versetzt und mit *Bartonella henselae* (1 x 10¹/ml) inokuliert. Das bakterielle Wachstum wurde bestimmt, indem in 24-stündigen Intervallen die optische Dichte (OD_{600 nm}) gemessen wurde (A, B). Die Anzahl der lebensfähigen Bakterien (CFU/ml) wurde parallel bestimmt, indem serielle Verdünnungen auf CBA ausplattiert wurden (C, D).

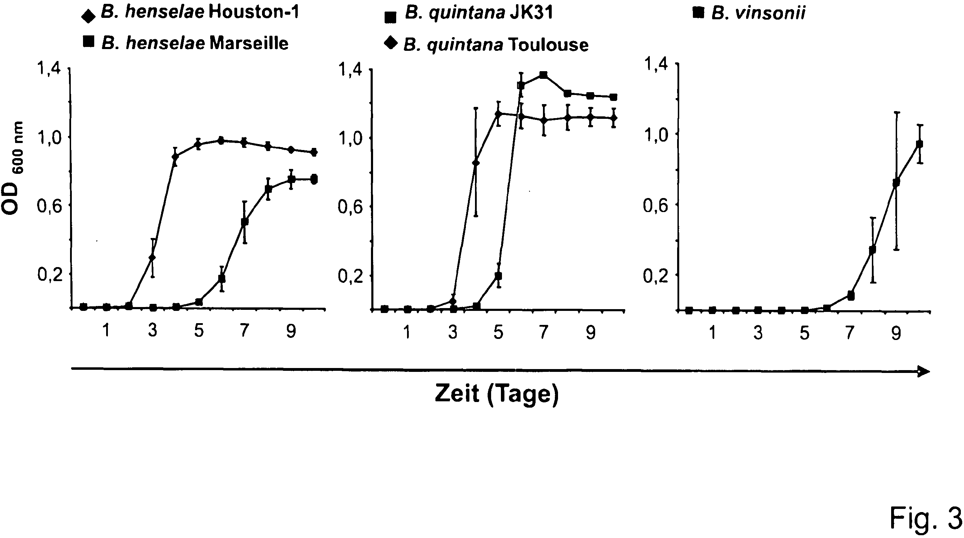

- Fig.3:: Wachstumskurven von verschiedenen *Bartonella-Stämmen* in Schneider's Medium. Schneider's Medium mit 10 Vol.-% FCS wurde mit *Bartonella henselae* Marseille, *Bartonella henselae* Houston-Einst, *Bartonella quintana* Toulouse, *Bartonella* JK31 und *Bartonella vinsonii* (1 x 10⁵ Bakterien/ml) inokuliert und das bakterielle Wachstum wurde bestimmt, indem in 24-stündigen Intervallen die optische Dichte (OD_{600 nm}) gemessen wurde. Festzustellen ist, dass alle Stämme in diesem Medium ein starkes Wachstum zeigen, obwohl die Intervalle vor dem Beginn des logarithmischen Wachstums (Lag-Phase) und die maximale optische Dichte unterschiedlich waren.

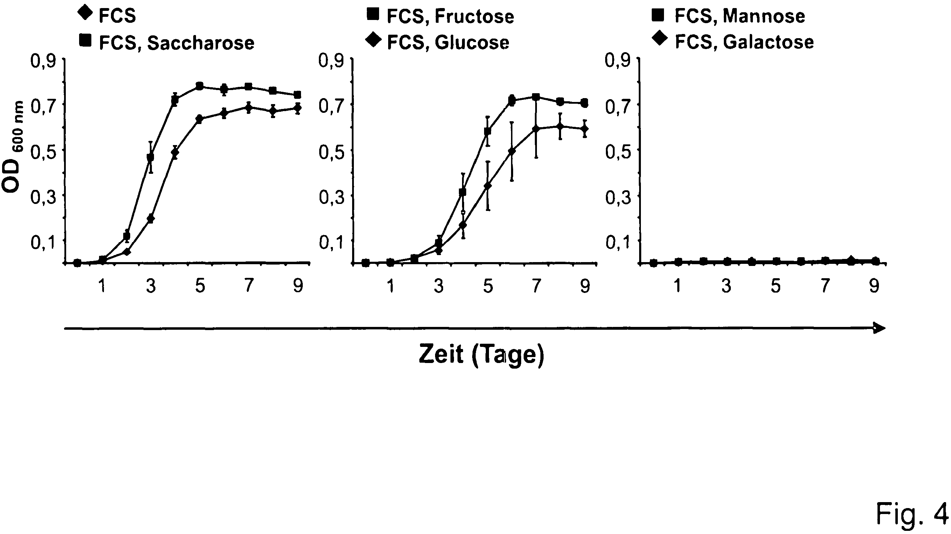

- Fig. 4:: Verbesserung des Schneider's Medium durch Zugabe von verschiedenen Zuckern. Schneider's Medium enthaltend 10 Vol.-% FCS wurde mit 5% (w/v) Saccharose, Fructose, Glucose, Mannose oder Galactose versetzt und mit *Bartonella henselae* (1 x 10⁶/ml) versetzt. Das bakterielle Wachstum wurde bestimmt, indem die optische Dichte (OD_{600 nm}) in 24-stündigen Intervallen gemessen wurde. Festzustellen ist, dass die höchsten Wachstumsraten und die maximalen optischen Dichten mit Schneider's Medium erzielt wurden, das 10 Vol.-% FCS und 5% (w/v) Saccharose enthielt.

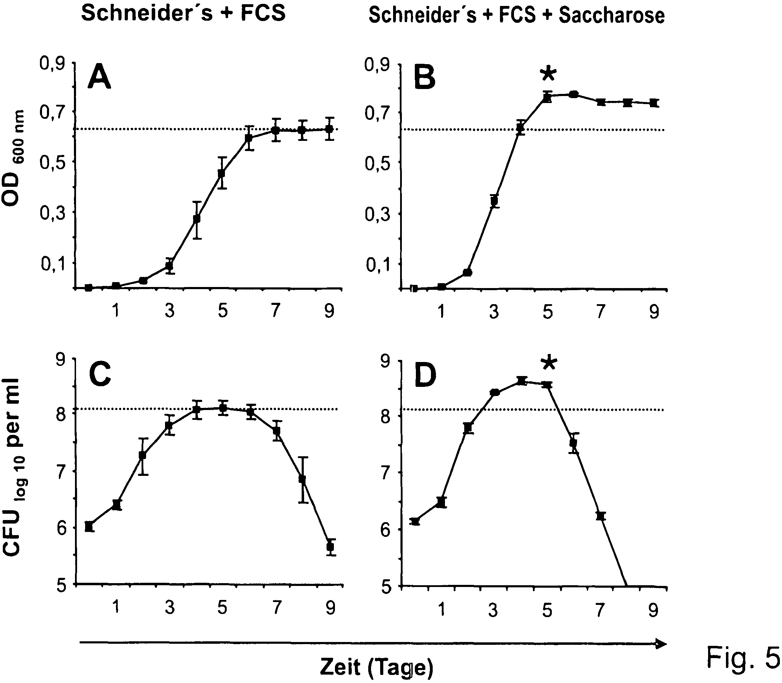

- Fig. 5:: Vergleich der Wachstumsraten in Schneider's Medium enthaltend 10 Vol.-% FCS in Abhängigkeit der Zugabe von Saccharose. Die Medien wurden mit *Bartonella henselae* (1 x 10⁶/ml) inokuliert und das bakterielle Wachstum wurde bestimmt, indem in 24-stündigen Intervallen entweder die optische Dichte (OD_{600 nm}; A, B) gemessen wurde, oder indem die Anzahl von lebensfähigen Bakterien (CFU/ml) quantifiziert wurde (C, D). Festzuhalten ist, dass die bakteriellen Wachstumsraten und die maximale Anzahl von lebensfähigen Bakterien höher sind in Gegenwart von Saccharose. Signifikanter Unterschied der maximalen OD (p = 0,001) oder CFU (p = 0,004) im Vergleich mit Medium ohne Saccharose (5. Tag).

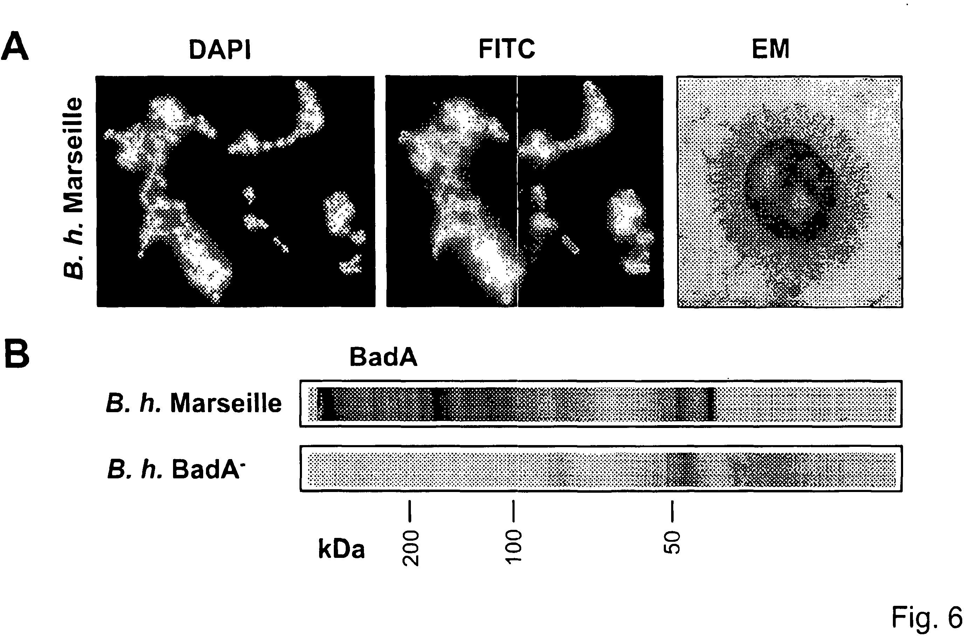

- Fig. 6:: BadA-Expression von *Bartonella henselae,* das in Schneider's basiertem *Bartonella*-Wachstumsmedium wächst. (A) *Bartonella henselae* Marseille wurde in Schneider's Medium enthaltend 10 Vol.-% FCS und 5% (w/v) Saccharose wachsen gelassen und die BadA-Expression wurde durch BadA-Immunfärbung und Elektronenmikroskopie (lange "haarige" Strukturen) analysiert. (B) Detektion von BadA in Flüssigkulturüberständen von *Bartonella henselae* Marseille durch BadA-Immunblotting. *Bartonella-henselae-*BadA⁻ wurde als Negativkontrolle verwendet.

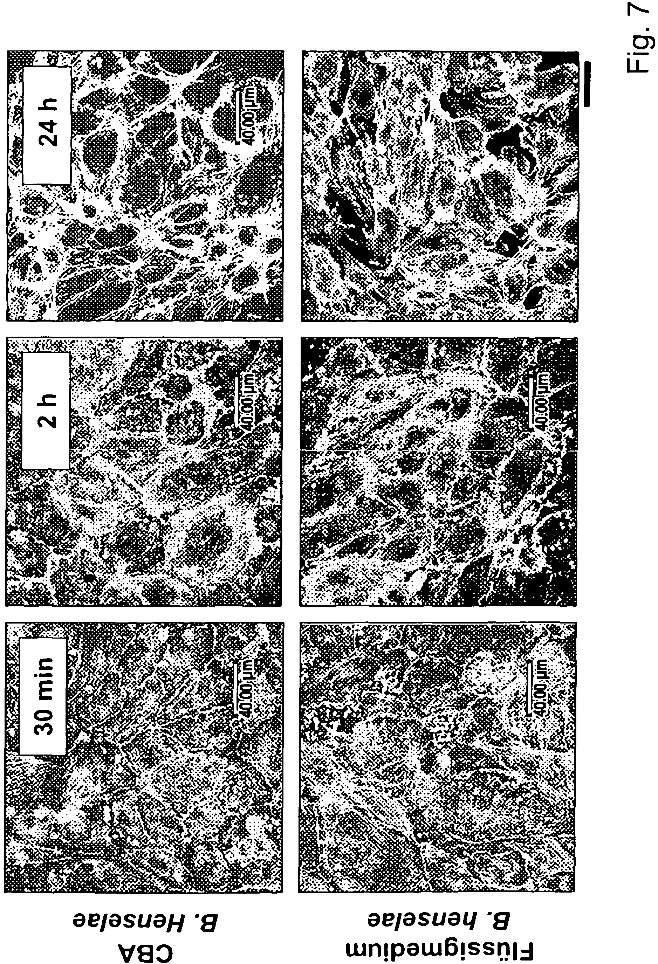

- Fig. 7:: Wirtszelladhäsion und Invasion von *Bartonella henselae,* das in Schneider's basiertem Bartonella-Wachstumsmedium gewachsen ist. HUVECs wurden mit *Bartonella henselae* infiziert, das entweder auf CBA oder in Schneider's Medium enthaltend 10 Vol.-% FCS und 5% (w/v) Saccharose gewachsen ist. Extra- und intrazelluläre Bakterien wurden mit CLSM 30 Minuten, 2 Stunden und 24 Stunden nach der Infektion visualisiert. Filamentöses Actin wurde gefärbt mit TRITC-markiertem Phalloidin. Maßstabsbalken: 40 µm.

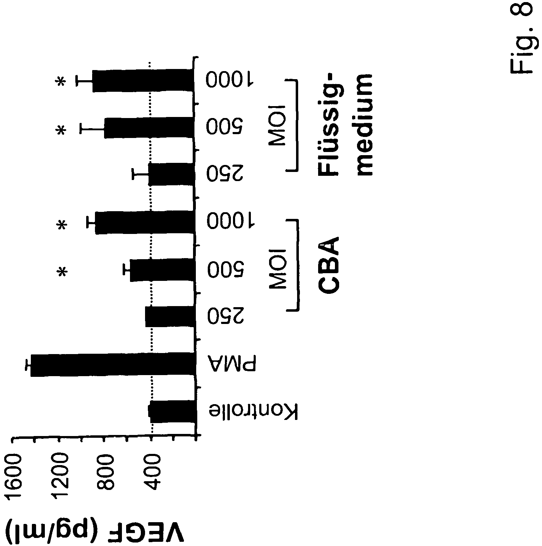

- Fig. 8:: Induktion der VEGF-A-Sekretion durch *Bartonella henselae,* das in Schneider's Medium gewachsen ist. HeLa 299-Zellen wurden mit *Bartonella henselae* infiziert, das entweder auf CBA oder in Schneider's Medium enthaltend 10 Vol.-% FCS und 5% (w/v) Saccharose gewachsen ist (MOI: 250, 500 und 1000). PMA (25 ng/ml) wurde als Positivkontrolle verwendet. VEGF-A wurde aus Zellkulturüberständen 24 Stunden nach der Infektion durch ELISA quantifiziert. Festzustellen ist, dass keine signifikanten Unterschiede in der Fähigkeit die VEGF-Sekretion zu triggern, zwischen den Bakterien festgestellt werden konnten, die auf Agar oder in Flüssigkultur gewachsen sind. Kontrolle: nicht infizierte Zellen. Signifikanter Unterschied im Vergleich zu der Kontrolle (p < 0,05).

### Ausführungsbeispiele

### 1. Material und Methoden

### 1.1 Bakterienstämme und Wachstumsbedingungen

*Bartonella henselae* Marseille (Drancourt, M. et al. (1996), New serotype of Bartonella henselae in endocarditis and cat-scratch disease. Lancet 347:441-443), *Bartonella henselae* BadA⁻ (keine Expression des Adhäsin A; (Riess, T. et al. (2004), Bartonella adhesion A mediates a proangiogenic host cell response. J. Exp. Med. 200:1267-1278), *Bartonella henselae* Houston-1 (ATCC 49882), *Bartonella quintana* Toulouse (Sammlung des Pasteur Instituts, Paris, Frankreich), *Bartonella quintana* JK31 (Zhang, P. et al. (2004), A family of variably expressed outer-membrane proteins (Vomp) mediates adhesion and autoaggregation in Bartonella quintana. Proc. Natl. Acad. Sci. U.S.A. 101:13630-13635) und *Bartonella vinsonii* subsp. *Berkhoffii* (ATCC 51672) wurden routinemäßig auf Columbiablutagar (CBA) in einer feuchten Atmosphäre bei 37°C und 5% CO₂ wachsengelassen. *Bartonella henselae* BadA⁻ wurde in Gegenwart von 25 µg/ml Kanamycin wachsengelassen. Sofern nicht anders angegeben, wurde *Bartonella henselae* Marseille in allen Experimenten verwendet. Zur Herstellung von Bakterienstammlösungen wurden die Bakterien von den Agarplatten nach 5 Tagen der Kultivierung geerntet und in Luria Bertani Medium (LB) enthaltend 20% Glycerol eingefroren.

### 1.2 Wachstum von Bartonella spp. in Flüssigmedium

Die Bakterien wurden in Flüssigmedium wachsengelassen, indem Zellkulturflaschen mit konstantem langsamem Schütteln (60 rpm/min) in einer feuchten Atmosphäre bei 37°C und 5 % CO₂ wachsengelassen wurden. Die folgenden Flüssigmedien wurden hinsichtlich des Wachstums von *Bartonella henselae* getestet. Hirn/Herz-Medium (BH), LB-Medium, *Bartonella* alpha-Proteobakterienwachstumsmedium (BAPGM), versetzt mit 10 Vol.-% fetalem Kälberserum (FCS; Sigma-Aldrich, München, Deutschland) anstelle von 5 Vol.-% defibriniertem Schafsblut), Endothelzellwachstumsmedium enthaltend Supplement-Mischung (Promocell, Heidelberg, Deutschland) und Schneider's Medium. In einigen Experimenten wurden die Flüssigmedien mit 10 Vol.-% FCS versetzt. Die Flüssigmedien wurden mit 1 x 10⁵ oder mit 1 x 10⁶ Bakterien/ml inokuliert und die jeweiligen optischen Dichten (OD_{600 nm}) wurden in 24-stündigen Intervallen in Dreifachmessungen bestimmt. Zusätzlich wurden die Anzahlen von kultivierten lebensfähigen Bakterien bestimmt, indem serielle Verdünnungen auf CBA-Platten ausplattiert wurden, gefolgt von einer Inkubationsdauer von 3 Wochen.

Schneider's Medium (Schneider's Drosophila Pulvermedium, revised, Serva, Heidelberg, Deutschland, Katalognr. 47521) setzt sich wie folgt zusammen:

**Tabelle 1: Zusammensetzung des Schneider's Medium**

| **Komponente** | **Konzentration (mg/l)** |
|---|---|
| Gesamtgewicht | 25.750 |
| ohne NaHCO₃ | |
| ohne CaCl₂ | |
| **Anorganische Salze** | |
| CaCl₂ wasserfrei | 600 |
| KCl | 1.600 |
| KH₂PO₄ | 450 |
| MgSO₄ 7H₂O | 3.700 |
| NaCl | 2.100 |
| NaHCO₃ | 400 |
| Na₂HPO₄ | 700 |
| **Aminosäure** | |
| (beta)-Alanin | 500 |
| L-Arginin | 600 |
| Asparaginsäure | 400 |
| L-Cystin | 60 |
| L-Cystin 2HCl | 21,7 |
| L-Glutamin | 1.800 |
| Glutaminsäure | 800 |
| Glycin | 250 |
| L-Histidin | 400 |
| L-Isoleucin | 150 |
| L-Leucin | 150 |
| L-Lysin HCl | 1.650 |
| L-Methionin | 150 |
| L-Prolin | 1.750 |
| L-Serin | 250 |
| L-Threonin | 350 |
| L-Tryptophan | 100 |
| L-Tyrosin | 500 |
| L-Valin | 300 |
| **Andere Komponenten** | |
| Apfelsäure | 60 |
| Bernsteinsäure | 600 |
| Fumarsäure | 60 |
| Glucose | 2.000 |
| Hefeextrakt | 2.000 |
| (alpha)-Ketoglutarsäure | 350 |
| (alpha)-Trehalose | 2.000 |

Das Medium wurde gemäß den Angaben des Herstellers präpariert:
Für 1 1 Medium werden ca. 800 ml destilliertes Wasser in einen 1-Liter Messkolben gegeben. 24,45 g des Pulvermediums werden unter Rühren hinzugegeben. Zum besseren Auflösen kann die Lösung auf 35°C erwärmt werden. 0,4 g Natriumbicarbonat und 0,6 g Calciumchlorid in jeweils ca. 10 ml dest. Wasser lösen und langsam unter Rühren dem Medium zufügen. pH-Wert von 6,5 unter Rühren mit 1 N NaOH oder 1 N HCl einstellen. Mit dest. Wasser auf 11 auffüllen, mit 10% FCS versetzen und steril filtrieren.

Um hinsichtlich optimalen bakteriellen Wachstums zu testen, wurden 5% (w/v) entweder Saccharose (Riedel de Haën, Seelze, Deutschland), Glucose (Sigma), Fructose (Merck, Darmstadt, Deutschland), Galactose (Merck) oder Mannose (Sigma) hinzugegeben.

### 1.3 Elektronenmikroskopie

Schneider's Medium enthaltend 10 Vol.-% FCS wurde mit *Bartonella henselae* Marseille (1 x 10⁶/ml) inokuliert. Nach 4 Tagen des Wachstums wurde die Bakterienkultur mit 2,5% Glutaraldehyd in Phosphat gepufferter Saline (PBS) für 15 Minuten bei 37°C fixiert und 24 Stunden bei 4°C gelagert. Die fixierten Zellen wurden in 2% Agarose eingebettet und kubische Blöcke von etwa 1 mm Länge wurden herausgeschnitten. Nach der Postfixierung mit 1% Osmium Tetroxid in 100 mM PO₄-Puffer, pH 7,2, für eine Stunde auf Eis, wurden diese Blöcke mit bidestilliertem Wasser gespült, mit 1%-igem wässrigen Uranylacetat für eine Stunde bei 4°C behandelt, durch abgestufte Verdünnungen von Äthanol dehydriert und in Epon eingebettet. Die Schnitte wurden mit 1% wässrigem Uranylacetat und Bleizitrat gefärbt und in einem Philips CM10 Elektronenmikroskop bei 60kV unter Verwendung einer 30 µm Objektivapertur analysiert.

### 1.4 Präzipitation von Proteinen von Bartonella henselae aus dem Überstand der Flüssigkultur

10 ml Schneider's Medium versetzt mit 10 Vol.-% FCS wurden mit *Bartonella henselae* (1 x 10⁷/ml) inokuliert und für 10 Tage inkubiert. Die Bakterienkulturen wurden für 15 Minuten bei 3.000 g zentrifugiert und die Proteine wurden aus dem Überstand durch Zugabe von 10% Trichloressigsäure für 1 Stunde auf Eis, gefolgt von einer Zentrifugation für 20 Minuten bei 20.000 g und 4°C präzipitiert. Das resultierende Pellet wurde dreimal in 1 ml eiskaltem Aceton resuspendiert, für 20 Minuten auf Eis inkubiert und für 15 Minuten bei 20.000 g und 4°C zentrifugiert. Schließlich wurde das resultierende Pellet in 700 µl destilliertem Wasser resuspendiert und bei -20°C für die weitere Verwendung gelagert.

### 1.5 Immunblotting

BadA wurde in der Fraktion des präzipitierten Proteins der Flüssigkulturüberstände von *Bartonella henselae* durch Immunblotting detektiert. Nach der Zugabe von Probenpuffer, der Natriumdodecylsulfat (SDS) enthielt, wurden die Proteine durch 12%-ige SDS-Polyacrylamidgel-Elektrophorese aufgetrennt und auf Nitrocellulosemembranen (Schleicher und Schuell, Dassel, Deutschland) transferiert. Die Membranen wurden für eine Stunde in 5% Magermilchpulver gelöst in 10 mM Tris pH 7,4, 0,15 M NaCl und 0,2% Tween 20 gelöst, gefolgt von einer Inkubation mit spezifischem BadA-Antikörper (Riess, T. et al. (2004), Bartonella adhesion A mediates a proangiogenic host cell response. J Exp.Med.200:1267-1278) über Nacht. Meerrettichperoxidase konjugierte sekundäre Antikörper wurden für die Detektion von Anti-BadA IgG verwendet, und die Signale wurden mit 3,3'-Diaminobenzidin-Tetrahydrochlorid (DAB; Applichem, Darmstadt, Deutschland) visualisiert.

### 1.6 Kultivierung und Infektion der Wirtszellen

Humane Endothelzellen aus der Nabelvene (HUVECs) wurden in Endothelzellwachstumsmedium (PromoCell, Heidelberg, Deutschland) kultiviert, wie beschrieben; vgl. Riess, T. (2007), Analysis of Bartonella adhesin A expression reveals differences between various B. henselae strains. Infect. Immun. 75:35-43. Für die Experimente zur Adhärenz und Invasion wurden 1 x 10⁵ Zellen pro Vertiefung auf Collagen G-beschichtete Deckgläschen am Tag vor dem Experiment ausgesät. Die Zellen wurden mit *Bartonella henselae* infiziert, die entweder in Flüssigmedium (Schneider's mit 10 Vol.-% FCS und 5% (w/v) Saccharose) gewachsen sind, oder mit bakteriellen Stammlösungen, die auf CBA mit einer "multiplicity of infection" (MOI) von 100. Nach 30 Minuten (Adhäsion) 2 Stunden und 24 Stunden (Invasion) wurden die Deckgläschen dreimal mit vorgewärmtem Endothelzellmedium gewaschen und in 3,75% Paraformaldehyd (PFA) fixiert.

Die HeLa-Zellen wuchsen in RPMI 1640, das mit 10% FCS und 2 mM Glutamin versetzt war. Für die Infektionsexperimente wurden 1 x 10⁵ Zellen pro Vertiefung in Zellkulturschalen mit 24 Vertiefungen am Tag vor dem Experiment ausgesät. 2 Stunden vor der Infektion wurde das Medium durch Medium ohne Antibiotika und FCS ausgetauscht, um die nicht spezifische Produktion von maskulärem endothelialem Wachstumsfaktor (VEGF) zu vermeiden. Die Zellen wurden mit *Bartonella henselae* infiziert, die entweder in Flüssigmedium (Schneider's mit 10 Vol.-% FCS und 5 Gew.-% Saccharose) wuchsen, oder mit bakteriellen Stammlösungen, die auf CBA wuchsen (MOI: 250, 500 und 1000). Die Bakterien wurden auf die kultivierten Zellen durch Zentrifugation für 5 Minuten bei 300 g. sedimentiert. 25 ng/ml Phorbol-12-Myristat-13-Acetat (PMA, Sigma-Aldrich) wurden als Positivkontrolle verwendet, um die Sekretion von VEGF-A zu induzieren. Die Überstände für VEGF-A-ELISAs wurden nach 24 Stunden entnommen und kurz zentrifugiert, um unlösliche Partikel abzutrennen.

### 1.7 Quantifizierung der VEGF-A-Sekretion

Die VEGF-A-Konzentrationen in den Zellkulturüberständen wurden quantifiziert, indem ein ELISA-Kit für humanen VEGF-A₁₆₅ (R&D Systems, Wiesbaden, Deutschland) gemäß der Anweisungen des Herstellers verwendet wurde.

### 1.8 Immunfärbung und Confokale Laserscanning-Mikroskopie (CLSM)

*Bartonella henselae* wurde in PBS resuspendiert, auf Glasträgern trocknengelassen und mit 3,75% PBS-gepuffertem PFA fixiert. Die Bakterien wurden gefärbt, wie zuvor bei der Verwendung von BadA spezifischen primären Antikörpern und Cyaninfarbe (Cy)2-konjugierten Sekundärantikörpern beschrieben (Dianova, Hamburg, Deutschland); vgl. Riess, T. et al. (2004), Bartonella adhesin A mediates a proangiogenic host cell response, J. Exp. Med. 200: 1267-1278). Die bakterielle DNA wurde mit 4',6-Diamidin-2'-Phenylindol-Dihydrochlorid (DAPI; Merck) gefärbt. Die Träger wurden mit einem Leica DMRE Fluoreszenzmikroskop analysiert, das mit einer Spot RT Monochrom-Digitalkamera und der dazugehörigen Spot Advanced Software versehen war (Visitron, Puchheim, Deutschland).

Für die mikroskopische Analyse der Wirtszelladhäsion und Invasion wurde eine differenzielle Färbung von extra- und intrazellulärem *Bartonella henselae* durchgeführt, wie zuvor beschrieben, mit wenigen Modifizierungen; vgl. Kempf, V.A. et al. (2000), Interaction of Bartonella henselae with endothelial cells results in rapid bacterial rRNA synthesis and replication. Cell. Microbiol. 2:431-441. Kurz zusammengefasst, PFA-fixierte Zellen wurden in PBS zu Beginn und nach jedem Inkubationsschritt gewaschen. Nicht spezifische Bindung von Antikörpern wurde mit 0,2% Rinderserumalbumin in PBS für 15 Minuten blockiert. Die Zellen wurden zuerst mit BadA-spezifischen primären Antikörpern (Riess, T. et al. (2004) Bartonella adhesion A mediates a proangiogenic host cell response. J. Exp. Med. 200:1267-1278) gefolgt von Cy2-konjugierten Sekundärantikörpern (Dianova) inkubiert. Als Nächstes wurden die Zellen durch Inkubation mit 0,2% Triton X-100 in PBS für 15 Minuten permeabilisiert und mit den BadA-spezifischen -Antikörpern gefolgt von Cy5-konjugierten Sekundärantikörpern (Dianova) inkubiert. Das Actin wurde mit Tetramethyl-Rohdamin-Isothiocyanit (TRITC) markiertem Phalloidin (Sigma-Aldrich) gefärbt. Die Träger wurden unter Verwendung von Einbettungsmedium eingebettet und mit einem Leica DM IRE 2 confokalem Laserscanningmikroskop analysiert. Die Aufnahmen wurden digital mit Adobe Photoshop 7.0 (Adobe Systems, Mountain View, CA, USA) digital verarbeitet.

### 1.9 Statistische Analyse

Sämtliche Experimente wurden in Dreifachansätzen durchgeführt. Die Experimente wurden wiederholt und ergaben vergleichbare Ergebnisse.

### 2. Ergebnisse

### 2.1 Entwicklung eines flüssigen Wachstumsmediums für Bartonella henselae

Bei Experimenten zur Interaktion von *Bartonella henselae* mit Insektenzellen (Schneider-Zellen von *Drosophila melanogaster;* vgl. Schneider, I. (1972), Cell lines derived from late embryonic stages of Drosophila melanogaster. J. Embryol. Exp. Morphol. 27:353-365.) wurde von den Erfindern festgestellt, dass *Bartonella henselae* in dem verwendeten Zellkulturmedium (Schneider's Drosophila Pulvermedium (Serva) versetzt mit 10 Vol.-% FCS und 2 mM Glutamin) gut wächst. Da bislang kein einfach herzustellendes flüssiges Wachstumsmedium für *Bartonella* spp. beschrieben ist, wurde das Schneider's Medium für die Verwendung als Wachstumsmedium für *Bartonella* weiter evaluiert.

Zunächst wurde das Wachstum von *Bartonella henselae* in Schneider's Medium, das mit FCS versetzt war, mit reinem Schneider's Medium verglichen. Beide Medien wurden mit *Bartonella henselae* (1 x 10⁵/ml) inokuliert und für 13 Tage inkubiert. Das bakterielle Wachstum wurde durch Messung der optischen Dichte (OD_{600 nm}) bestimmt, wobei sich eine typische Wachstumskurve mit einer Lag-Phase, einer logarithmischen Wachstumsphase und einer stationären Phase bei Verwendung des FCS-versetzten Mediums (Fig. 1A) ergab, während reines Schneider's Medium das Wachstum von *Bartonella henselae* nicht unterstützte (Fig. 1B).

Da die Zugabe von FCS zu Schneider's Medium für das bakterielle Wachstum wichtig war, wurde als Nächstes das Wachstum von *Bartonella* in zwei mikrobiologischen Standardmedien, (LB, BH) und in Endothelzellwachstumsmedium, untersucht, die allesamt mit 10 Vol.-% FCS versetzt waren. Bemerkenswerterweise unterstützte keines dieser Medien das Wachstum von *Bartonella henselae* (Fig. 1C-E).

Als Nächstes wurde das Wachstum von *Bartonella henselae* in Schneider's Medium mit dem Wachstum in BAPGM (Maggi, R.G. et al. (2005), Novel chemically modified liquid medium that will support the growth of seven Bartonella species. J. Clin. Microbiol. 43:2651-2655), jeweils versetzt mit 10 Vol.-% FCS. Beide Medien wurden mit *Bartonella henselae* (1 x 10⁵/ml) inokuliert und für 13 Tage inkubiert. Das Wachstum der Bakterien wurde bestimmt, indem die optische Dichte gemessen wurde (Fig. 2A, B) und die Anzahl von lebensfähigen Bakterien (CFU/ml) zu den gleichen Zeitpunkten gezählt wurden (Fig. 2C, D). Die OD-Werte zeigten einen entsprechenden Verlauf in beiden Medien, obwohl die finale OD in BAPGM mit 10% FCS im Vergleich zu Schneider's mit 10 Vol.-% FCS höher war (0,84 vs. 0,60).

Die Bestimmung der Bakterienzahlen zeigte in beiden Medien eine Lag-Phase von 2-3 Tagen gefolgt von einer exponentiellen Wachstumsphase. Die maximale Bakterienzahl war in Schneider's Medium enthalten 10 Vol.-% FCS im Vergleich zu BAPGM mit 10 Vol.-% FCS leicht erhöht (1,9 x 10⁸ vs. 1,2 x 10⁸ CFU/ml). Nach der exponentiellen Wachstumsphase nahm die Anzahl von lebensfähigen Bakterien rasch ab (Todphase) mit keiner nachweisbaren stationären Phase; ein solches Fehlen einer stationären Phase wurde für *Bartonella henselae* bereits vorher beschrieben; vgl. Maggi, R.G. (2005), Novel chemically modified liquid medium that will support the growth of seven Bartonella species. J. Clin. Microbiol. 43:2651-2655.

### 2.2 Wachstum von verschiedenen Bartonella spp. in Schneider's Medium enthaltend 10 Vol.-% FCS

Als Nächstes wurde das Wachstum von anderen humanmedizinischen und veterinärmedizinisch wichtigen *Bartonella* spp. Analysiert, nämlich von *Bartonella henselae* Marseille, *Bartonella henselae* Houston-1, *Bartonella quintana* Toulouse, *Bartonella quintana* JK31 und *Bartonella vinsonii.* Erneut wurde Schneider's Medium versetzt mit 10 Vol.-% FCS mit 1 x 10⁵ Bakterien/ml von jedem Stamm inokuliert und für 10 Tage inkubiert (Fig. 3). Sämtliche fünf Stämme zeigten bakterielles Wachstum, wobei die Zeiten, bis diese die jeweilige exponentielle Phase und die maximalen optischen Dichten erreichten, unterschiedlich waren (Lag-Phase von zwei Tagen für *Bartonella henselae* Houston-1, drei Tage für *Bartonella quintana* Toulouse, vier Tage für *Bartonella quintana* JK31, fünf Tage für *Bartonella henselae* Marseille und sechs Tage für *Bartonella vinsonii*).

Der am besten wachsende Stamm war *Bartonella quintana* JK31 (OD_{600 nm}: 1,37 am 7. Tag), die am langsamsten wachsenden Stämme waren *Bartonella henselae* Marseille (OD_{600 nm}: 0,76 am 9. Tag) und *Bartonella vinsonii* (OD_{600 nm}: 0,95 am 10. Tag). Die Daten zeigen, dass selbst innerhalb einer bestimmten Spezies die Generationszeiten und die maximalen Wachstumsraten signifikant unterschiedlich sein können (z.B. für *Bartonella henselae* Houston-1 und *Bartonella henselae* Marseille).

### 2.3 Verbesserung des Schneider's basierenden Bartonella Wachstummediums

Während verschiedene Methoden zur genetischen Manipulation von *Bartonella henselae* evaluiert wurden, stellten die Erfinder fest, dass die Zugabe von Saccharose zu Schneider's Medium mit 10 Vol.-% FCS in einem noch schnelleren Bakterienwachstum resultiert. Deshalb wurde systematisch der Einfluss von verschiedenen Kohlenhydraten auf die Wachstumsraten von *Bartonella henselae* untersucht.

Zu diesem Zwecke wurde Schneider's Medium enthaltend 10 Vol.-% FCS mit 5% (w/v) Saccharose, Fructose, Glucose, Mannose oder Galactose versetzt. Die Medien wurden mit *Bartonella henselae* (1 x 10⁶/ml) inokuliert und das Bakterienwachstum wurde durch Messung der optischen Dichte bestimmt (Fig. 4).

Die Daten ergaben, dass die Zugabe von Saccharose die Wachstumsraten von *Bartonella henselae* erhöhten, während Fructose keinen signifikanten Effekt zeigte. Bemerkenswerterweise unterdrückte die Zugabe von Mannose oder Galactose das Wachstum von *Bartonella henselae* vollständig, möglicherweise aufgrund von toxischen Effekten.

Da signifikante Unterschiede zwischen den optischen Dichten und der Zahl von lebensfähigen Bakterien beobachtet wurden (siehe Fig. 2), wurden beide Werte auch in Schneider's Medium enthaltend 10 Vol.-% FCS entweder mit oder ohne 5% (w/v) Saccharose bestimmt (Fig. 5). In Übereinstimmung mit der optischen Dichte war die maximale Bakterienzahl ~3,3-fach höher, wenn Saccharose hinzugegeben wurde. Erneut wurde eine schnelle Abnahme der Anzahl von lebenden Bakterien (Todphase) nach der exponentiellen Wachstumsphase beobachtet. Mit diesen Daten, der maximalen Wachstumsphase zwischen dem ersten Tag und zweiten Tag, konnte die Generationsdauer von ungefähr 5,6 Stunden [Berechnung der Generationsdauer: 1 x Ig2(t-t₀)/IgN-IgN₀] in Schneider's Medium enthaltend 10 Vol.-% FCS und 5% (w/v) Saccharose berechnet werden.

### 2.4 BadA Expression von Bartonella henselae, die in Schneider's basiertem Bartonella Wachstumsmedium gewachsen sind

Da bekannt ist, dass die Expression des *Bartonella* Adhesin A (BadA), einem wichtigen Pathogenitätsfaktor von *Bartonella henselae,* von den Kultivierungsbedingungen abhängt, wurde das Wachstum von *Bartonella henselae* Marseille in dem erfindungsgemäßen Flüssigkeitsmedium hinsichtlich der Expression von BadA analysiert. Die Immunfluoreszenz unter Verwendung spezifischer BadA-Antikörper und die Elektronenmikroskopie (Fig. 6A) zeigte eine starke Oberflächenexpression. BadA wurde ebenfalls in den Überständen der *Bartonella henselae* Kulturen detektiert, die in Schneider's Medium enthaltend 10 Vol.-% FCS gewachsen sind, und zwar durch Immunblotting mit spezifischen Anti-BadA-Antikörpern (Fig. 6B). Hier waren die typischen leiterähnlichen Muster der BadA-Banden detektierbar, von denen bekannt ist, dass diese in *Bartonella henselae* BadA⁻-Mutanten abwesend sind. Entsprechende Ergebnisse wurden erhalten, wenn Saccharose zu dem Medium hinzugegeben wurde (Daten nicht gezeigt).

### 2.5 Funktionelle Analyse von Bartonella henselae, die in Schneider's basiertem Bartonella-Wachstumsmedium gewachsen sind

*Bartonella henselae* adhäriert an und invadiert in Endothelzellen und induziert die Sekretion von vaskulärem Endothelzellwachstumsfaktor A VEGF-A; (Kempf, V.A. et al. (2001), Evidence of a leading role for VEGF in Bartonella henselae-induced endothelial cell proliferations. Cell. Microbiol. 3:623-632). Deshalb wurde die Wirtszellinteraktion von *Bartonella henselae,* das auf CBA oder in Flüssigmedium gewachsen ist, analysiert. Zunächst wurden HUVECs mit *Bartonella henselae* infiziert, die entweder auf CBA oder in Schneider's Medium enthaltend 10 Vol.-% FCS und 5% (w/v) Saccharose gewachsen sind. Die Adhäsion und Inversion wurde 30 Minuten, 2 Stunden und 24 Stunden nach der Infektion mit CLSM analysiert (Fig. 7). Es konnte kein Unterschied in der Anzahl von adhärenten und intrazellulären *Bartonella henselae* beobachtet werden, unabhängig von den jeweiligen Wachstumsbedingungen.

Als nächstes wurde die Fähigkeit von *Bartonella henselae* zur Induktion der Sekretion von VEGF-A durch infizierte Wirtszellen analysiert. HeLa-Zellen wurden mit *Bartonella henselae* infiziert und die VEGF-A-Konzentrationen in den Überständen wurden 24 Stunden nach der Infektion verglichen (Fig. 8). Die Daten ergaben, dass vergleichbare Mengen von VEGF-A unter beiden experimentellen Bedingungen unabhängig von den Wachstumsbedingungen der Bakterien sekretiert wurden.

### 3. Fazit

Die Erfinder stellen erstmals eine Zusammensetzung zur Kultivierung von anspruchsvollen Bakterien bereit, die ein rasches Wachstum ermöglicht.

## Patentansprüche

1. Verwendung einer Zusammensetzung zur Kultivierung von Bakterien der Gattung *Bartonella,* **dadurch gekennzeichnet, dass** die Zusammensetzung modifiziertes Schneider's Drosophila Medium ("Schneider's Drosophila Medium revised") und Fetales Kälberserum (FCS) aufweist.

2. Verwendung nach Anspruch 1, **dadurch gekennzeichnet, dass** die Zusammensetzung folgende Bestandteile aufweist:
| Anorganische Salze, nämlich | | |
|---|---|---|
| | CaCl₂ (wasserfrei) [in mg/l H₂O]: | 600, |
| | KCl [in mg/l H₂O]: | 1600, |
| | KH₂PO₄ [in mg/l H₂O]: | 450, |
| | MgSO₄·7H₂O [in mg/l H₂O]: | 3700, |
| | NaCl [in mg/l H₂O]: | 2100, |
| | NaHCO₃ [in mg/l H₂O]: | 400, |
| | Na₂HPO₄ [in mg/l H₂O]: | 700, |
| | | |
| Aminosäuren, nämlich | | |
|---|---|---|
| | β-Alanin [in mg/l H₂O]: | 500, |
| | L-Arginin [in mg/l H₂O]: | 600, |
| | L-Asparaginsäure [in mg/l H₂O]: | 400, |
| | L-Cystein [in mg/l H₂O]: | 60, |
| | L-Cystein-2HCl [in mg/l H₂O]: | 21,7, |
| | L-Glutamin [in mg/l H₂O]: | 1800, |
| | L-Glutaminsäure [in mg/l H₂O]: | 800, |
| | Glycin [in mg/l H₂O]: | 250, |
| | L-Histidin [in mg/l H₂O]: | 400, |
| | L-Isoleucin [in mg/l H₂O]: | 150, |
| | L-Leucin [in mg/l H₂O]: | 150, |
| | L-Lysin·HCl [in mg/l H₂O]: | 1650, |
| | L-Methionin [in mg/l H₂O]: | 150, |
| | L-Prolin [in mg/l H₂O]: | 1750, |
| | L-Serin [in mg/l H₂O]: | 250, |
| | L-Threonin [in mg/l H₂O]: | 350, |
| | L-Tryptophan [in mg/l H₂O]: | 100, |
| | L-Tyrosin [in mg/l H₂O]: | 500, |
| | L-Valin [in mg/l H₂O]: | 300, |
| | | |
| andere Bestandteile, nämlich | | |
|---|---|---|
| | Apfelsäure [in mg/l H₂O]: | 60, |
| | Bernsteinsäure [in mg/l H₂O]: | 600, |
| | Fumarinsäure [in mg/l H₂O]: | 60, |
| | Glucose [in mg/l H₂O]: | 2000, |
| | Hefe-Extrakt [in mg/l H₂O]: | 2000, |
| | α-Ketoglutarsäure [in mg/l H₂O]: | 350, |
| | α-Trehalose [in mg/l H₂O]: | 2000, und |
| | | |
| Fetales Kälberserum (FCS) [in Vol.-%] | | 10. |

3. Verwendung nach einem der vorherigen Ansprüche, **dadurch gekennzeichnet, dass** die Zusammensetzung zusätzlich 0,5 bis 50% (w/v) Saccharose aufweist.

4. Verwendung nach Anspruch 3, **dadurch gekennzeichnet, dass** die Zusammensetzung 5% (w/v) Saccharose aufweist.

5. Verwendung nach einem der vorherigen Ansprüche, **dadurch gekennzeichnet, dass** die Zusammensetzung einen pH-Wert aufweist, der bei 5,5 bis 7,5 liegt.

6. Verwendung nach Anspruch 5, **dadurch gekennzeichnet, dass** die Zusammensetzung einen pH-Wert aufweist, der bei 6,5 liegt.

7. Verwendung nach einem der vorherigen Ansprüche, **dadurch gekennzeichnet, dass** die Zusammensetzung sterilisiert ist.

8. Verfahren zur Kultivierung von Bakterien der Gattung *Bartonella,* das folgende Schritte aufweist,
(1) Bereitstellung einer Zusammensetzung,
(2) Inokulieren der Zusammensetzung mit Bakterien der Gattung *Bartonella,*
(3) Inkubation der Zusammensetzung mit den Bakterien unter Kultivierungsbedingungen,
**dadurch gekennzeichnet, dass** als Zusammensetzung die Zusammensetzung der Verwendung nach einem der Ansprüche 1 bis 7 verwendet wird.

9. Verfahren nach Anspruch 8, **dadurch gekennzeichnet, dass** in Schritt (2) die Inokulation mit 1 x 10⁵ bis 1 x 10⁶ Bakterienzellen erfolgt.

10. Verfahren nach Anspruch 8 oder 9, **dadurch gekennzeichnet, dass** in Schritt (3) die Inkubation für 1 bis 20 Tage erfolgt, vorzugsweise bei 37°C erfolgt, und weiter vorzugsweise die Inkubation in einer Atmosphäre mit 5 Vol.-% CO₂ erfolgt.

## Claims

1. Use of a composition for the cultivation of bacteria of the genus *Bartonella,* **characterized in that** the composition comprises modified Schneider's Drosophila medium ("Schneiders Drosophila Medium revised") and fetal calf serum (FCS).

2. Use of claim 1, **characterized in that** the composition comprises the following components:
| Inorganic salts, namely | | |
|---|---|---|
| | CaCl₂ (anhydrous) [in mg/l H₂O]: | 600, |
| | KCl [in mg/l H₂O]: | 1600, |
| | KH₂PO₄ [in mg/l H₂O]: | 450, |
| | MgSO₄·7H₂O [in mg/l H₂O]: | 3700, |
| | NaCl [in mg/l H₂O]: | 2100, |
| | NaHCO₃ [in mg/l H₂O]: | 400, |
| | Na₂HPO₄ [in mg/l H₂O]: | 700, |
| amino acids, namely | | |
|---|---|---|
| | β-alanine [in mg/l H₂O]: | 500, |
| | L-arginine [in mg/l H₂O]: | 600, |
| | L-aspartic acid [in mg/l H₂O]: | 400, |
| | L-cysteine [in mg/l H₂O]: | 60, |
| | L-cysteine-2HCl [in mg/l H₂O]: | 21.7, |
| | L-glutamine [in mg/l H₂O]: | 1800, |
| | L-glutamic acid [in mg/l H₂O]: | 800, |
| | glycine [in mg/l H₂O]: | 250, |
| | L-histidine [in mg/l H₂O]: | 400, |
| | L-isoleucine [in mg/l H₂O]: | 150, |
| | L-leucine [in mg/l H₂O]: | 150, |
| | L-lysine·HCl [in mg/l H₂O]: | 1650, |
| | L-methionine [in mg/l H₂O]: | 150, |
| | L-proline [in mg/l H₂O]: | 1750, |
| | L-serine [in mg/l H₂O] | 250, |
| | L-threonine [in mg/l H₂O]: | 350, |
| | L-tryptophan [in mg/l H₂O]: | 100, |
| | L-tyrosine [in mg/l H₂O]: | 500, |
| | L-valine [in mg/l H₂O]: | 300, |
| | | |
| other components, namely | | |
|---|---|---|
| | malic acid [in mg/l H₂O]: | 60, |
| | succinic acid [in mg/l H₂O] | 600, |
| | fumaric acid [in mg/l H₂O]: | 60, |
| | glucose [in mg/l H₂O]: | 2000, |
| | yeast extract [in mg/l H₂O]: | 2000, |
| | α-ketoglutaric acid [in mg/l H₂O]: | 350, |
| | α-trehalose [in mg/l H₂O]: | 2000, and |
| fetal calf serum (FCS) [in vol.-%] | | 10. |

3. Use of any of the preceding claims, **characterized in that** the composition additionally comprises 0.5 to 50 % (w/v) sucrose.

4. Use of claim 3, **characterized in that** the composition comprises 5 % (w/v) sucrose.

5. Use of any of the preceding claims, **characterized in that** the composition comprises a pH value of 5.5 to 7.5

6. Use of claim 5, **characterized in that** the composition comprises a pH value of 6.5

7. Use of any of the preceding claims, **characterized in that** the composition is sterilized.

8. Method for the cultivation of bacteria of the genus *Bartonella,* comprising the following steps,
(1) providing a composition,
(2) inoculating the composition with bacteria of the genus *Bartonella,*
(3) incubating the composition with the bacteria under cultivation conditions, **characterized in that** the composition is the composition of the use of any of claims 1 to 7.

9. Method of claim 8, **characterized in that** in step (2) the inoculation is realized with 1 x 10⁵ to 1 x 10⁶ bacteria cells.

10. Method of claim 8 or 9, **characterized in that** in step (3) the incubation is realized for 1 to 20 days, preferably is realized at 37°C, and further preferably is realized in an atmosphere of 5 vol.-% CO₂.

## Revendications

1. Utilisation d'une composition pour la culture de bactéries du genre *Bartonella,* **caractérisée en ce que** la composition présente un milieu de drosophile de Schneider modifié (» Schneider's Drosophila Medium revised ») et un sérum foetal bovin (FCS).

2. Utilisation selon la revendication 1, **caractérisée en ce que** la composition présente les composants suivants :
| des sels anorganiques, à savoir | |
|---|---|
| CaCl₂ (anhydre) [en mg/l H₂O] : | 600, |
| KCl [en mg/l H₂O] : | 1600, |
| KH₂PO₄ [en mg/l H₂O] : | 450, |
| MgSO₄ . 7H₂O [en mg/l H₂O] : | 3700, |
| NaCl [en mg/l H₂O] : | 2100, |
| NaHCO₃ [en mg/l H₂O] : | 400, |
| Na₂HPO₄ [en mg/l H₂O] : | 700, |
| des acides aminés, à savoir | |
| β-alanine [en mg/l H₂O] : | 500, |
| L-arginine [en mg/l H₂O] : | 600, |
| acide L-aspartique [en mg/l H₂O] : | 400, |
| L-cystéine [en mg/l H₂O] : | 60, |
| L-cystéine . 2HCl [en mg/l H₂O] : | 21,7, |
| L-glutamine [en mg/l H₂O] : | 1800, |
| acide L-glutamique [en mg/l H₂O] : | 800, |
| glycine [en mg/l H₂O] : | 250, |
| L-histidine [en mg/l H₂O] : | 400, |
| L-isoleucine [en mg/l H₂O] : | 150, |
| L-leucine [en mg/l H₂O] : | 150, |
| L-lysine . HCl [en mg/l H₂O] : | 1650, |
| L-méthionine [en mg/l H₂O] : | 150, |
| L-proline [en mg/l H₂O] : | 1750, |
| L-sérine [en mg/l H₂O] : | 250, |
| L-thréonine [en mg/l H₂O] : | 350, |
| L-tryptophane [en mg/l H₂O] : | 100, |
| L-tyrosine [en mg/l H₂O] : | 500, |
| L-valine [en mg/l H₂O] : | 300, |
| d'autres composants, à savoir | |
| acide malique [en mg/l H₂O] : | 60, |
| acide succinique [en mg/l H₂O] : | 600, |
| acide fumarique [en mg/l H₂O] : | 60, |
| glucose [en mg/l H₂O] : | 2000, |
| extrait de levure [en mg/l H₂O] : | 2000, |
| acide α-cétoglutarique [en mg/l H₂O] : | 350, |
| α-tréhalose [en mg/l H₂O] : | 2000, et |
| sérum foetal bovin (FCS) [en % en vol.] | 10. |

3. Utilisation selon l'une des revendications précédentes, **caractérisée en ce que** la composition présente en outre 0,5 à 50 % (p/v) de saccharose.

4. Utilisation selon la revendication 3, **caractérisée en ce que** la composition présente 5 % (p/v) de saccharose.

5. Utilisation selon l'une des revendications précédentes, **caractérisée en ce que** la composition présente un pH qui est de 5,5 à 7,5.

6. Utilisation selon la revendication 5, **caractérisée en ce que** la composition présente un pH qui est de 6,5.

7. Utilisation selon l'une des revendications précédentes, **caractérisée en ce que** la composition est stérilisée.

8. Procédé de culture de bactéries du genre *Bartonella,* qui présente les étapes suivantes :
(1) mise à disposition d'une composition,
(2) inoculation de la composition avec des bactéries du genre *Bartonella,*
(3) incubation de la composition avec les bactéries dans des conditions de culture,
**caractérisé en ce que** l'on utilise comme composition, la composition de l'utilisation selon l'une des revendications 1 à 7.

9. Procédé selon la revendication 8, **caractérisé en ce qu'**à l'étape (2), l'inoculation s'effectue avec 1 10⁵ à 1 x 10⁶ bactéries.

10. Procédé selon la revendication 8 ou 9, **caractérisé en ce qu'**à l'étape (3), l'incubation s'effectue pendant 1 à 20 jours, de préférence, à 37° C et en outre, de préférence, l'incubation s'effectue dans une atmosphère à 5 % en vol. de CO₂.
